(19) Europäisches Patentamt
European Patent Office
Office européen des brevets

(11) **EP 4 538 391 A1**

(12) **EUROPEAN PATENT APPLICATION**
published in accordance with Art. 153(4) EPC

(43) Date of publication:
**16.04.2025 Bulletin 2025/16**

(21) Application number: 23742348.8

(22) Date of filing: **07.06.2023**

(51) International Patent Classification (IPC):
***C12Q 1/6883*** (2018.01)

(52) Cooperative Patent Classification (CPC):
**C12Q 1/6883;** C12Q 2600/156

(86) International application number:
**PCT/ES2023/070378**

(87) International publication number:
**WO 2023/237800 (14.12.2023 Gazette 2023/50)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **07.06.2022 ES 202230497**

(71) Applicants:
• **Baigene, S.L.**
  **01510 Vitoria-Gasteiz Alava (ES)**
• **Santxa Research S.L.U.**
  **01008 Vitoria-Gasteiz (ES)**

(72) Inventors:
• **YARZA, Izarbe**
  **01510 Vitoria-Gasteiz (Álava) (ES)**
• **CELORRIO, David**
  **01510 Vitoria-Gasteiz (Álava) (ES)**

• **AZNAR, Jose M.**
  **01510 Vitoria-Gasteiz (Álava) (ES)**
• **JORQUERA, Cristina**
  **01008 Vitoria-Gasteiz (Álava) (ES)**
• **SÁNCHEZ, Pello**
  **01008 Vitoria-Gasteiz (Álava) (ES)**
• **BEITIA, Maider**
  **01008 Vitoria-Gasteiz (Álava) (ES)**
• **DELGADO, Diego**
  **01008 Vitoria-Gasteiz (Álava) (ES)**
• **SÁNCHEZ, Mikel**
  **01008 Vitoria-Gasteiz (Álava) (ES)**

(74) Representative: **Pons**
  **Glorieta Rubén Darío 4**
  **28010 Madrid (ES)**

Remarks:
The complete document including Reference
Table(s) and the Sequence Listing(s) can be
downloaded from the EPO website

(54) **METHOD FOR OBTAINING USEFUL DATA FOR THE PREDICTION OF RISK OF FIBROSIS IN A SUBJECT**

(57) The present invention relates to an *in vitro* method of obtaining data useful for predicting a subject's risk of suffering fibrosis, being comprised in the field of personalized medicine. Specifically, said method is based on the analysis of a series of genetic polymorphisms of the subject, as well as other environmental data thereof, which together are useful and allow predicting a subject's risk of developing fibrosis.

EP 4 538 391 A1

**Description**

The present invention relates to an *in vitro* method of obtaining data useful for predicting a subject's risk of suffering fibrosis, being comprised in the field of personalized medicine. Specifically, said method is based on the analysis of a series of genetic polymorphisms, as well as other environmental data of the subject, which together are useful and allow predicting a subject's risk of developing fibrosis.

**BACKGROUND ART**

In general terms, fibrosis refers to the growth and abnormal accumulation of extracellular matrix components which causes tissue hardening or fibrosation, resulting in a partial or complete loss of function in the affected tissue or organ. The fibrotic disease comprises a wide range of clinical pathologies which are considered to be responsible for 45% of all deaths in the developed world.

Fibrotic pathologies can affect a large number of different organs and tissues; however, it is believed that their generation pathways are common in any organ. On the other hand, fibrotic pathology can occur after a surgical intervention. This condition, known as postoperative fibrosis, is characterized by an excessive proliferation of fibrotic and scar tissue generated after a surgical intervention in a patient, with the formation of more fibrous tissue than necessary, which usually has a negative impact on the patient's quality of life.

Fibrosis appears very frequently at the joint level, leading to the development of arthrofibrosis, with the subsequent pathological stiffening or hardening of a joint due to an exaggerated fibrotic response. For example, the appearance of fibrosis is a complication to be taken into account in surgical interventions performed on the joint due to its incidence: ranging from 1 to 15% of affected patients in knee arthroplasties, and between 4 and 38% in anterior cruciate ligament reconstruction.

Taking into account that in the US alone an estimated 85,000 new cases of arthrofibrosis, solely of the knee, may arise every year, and that 25% of them require additional surgery to try to re-establish proper knee movement, the implications not only at the level of worsened quality of life derived from less mobility, but also the social and economic repercussion of this type of pathology, can be estimated.

Various methods relating to the diagnosis or prognosis of fibrosis or similar conditions have been described: Patent application EP2428583A1 describes a method for determining the progress of a healing process in a subject, which comprises determining the expression level of at least one gene selected from the genes TFAP2A, EGFR, ILIA, IL1B, IL6, IL10, IL18, CD44, TNFRSF1A, HSPD1, MYC, CTGF, MMP7, MMP2, MMP9, MMP25, TGFA, TGFB2, EREG, FN1, HBEGF, NF1, SRC, EGF4, CDKN2A, PLAU, SPP1, ITGB2, BAX, MET, and PPAR, or a functionally equivalent variant of said genes, in a sample from said subject.

Document ES2422874B1 describes an *in vitro* method for the prognosis and/or diagnosis of severe liver fibrosis characterized by the detection of a series of genetic polymorphisms and the determination of clinical variables.

Document CA2805267A1 describes a method which comprises measuring the level of cadherin-11 in a sample obtained from a subject, and comparing same with control values, wherein a cadherin-11 level in the sample obtained from the subject greater than the control value indicates fibrosis or risk of developing fibrosis.

However, the described methods do not allow reliably predicting a subject's risk of suffering fibrosis, which hinders and limits decision-making or the adoption of preventive measures that can prevent this condition. Thus, in light of the prior art, there is a need to provide methods that are useful in predicting a subject's risk of suffering a fibrosis process and allow reliably determining the risk of suffering complications of this type.

**DESCRIPTION OF THE INVENTION**

The present invention discloses an *in vitro* method of obtaining data useful for predicting the risk of a subject of suffering fibrosis

The inventors develop the method based on the use of environmental variables (degree of obesity, use or non-use of platelet-rich plasma, and furthermore data relating to the variablesarea susceptible to suffering fibrosis and/or sex of the subject), together with genetic variables comprising the analysis of one or more genetic polymorphisms of genes MMP3, NEDD4, SMAD4, as well as their combinations with polymorphisms of genes IL6R, CTGF, IL-6, BMP4, The application of an algorithm that takes into account the preceding variables allows obtaining a fibrosis generation risk value for a subject.

In the scientific literature, there are association studies that separately determine the involvement of genetic and environmental factors in fibrotic events at the pulmonary level, hepatic level, joint level, or others. However, the algorithm of the invention takes into account genetic and environmental variables jointly, allowing a specific risk value to be calculated for each subject.

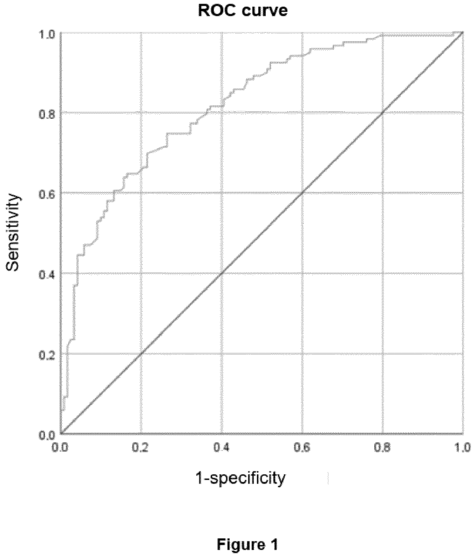

The inventors have observed that the application of this approach in patients has high sensitivity and specificity, with the method of the invention having a good predictive capacity, as shown by the ROC (Receiver Operating Characteristic) (Figure 1; Tables 7, 8, and 9).

The present invention therefore provides an innovative approach in the area of personalized medicine with multiple associated advantages: it allows obtaining data useful for knowing a patient's risk of suffering fibrosis with high sensitivity and specificity; it allows decision-making or the adoption of preventive measures that can prevent said condition, which in the event that the patient is to be subjected to a surgery, it can in turn facilitate proper recovery and/or future interventions to eliminate the fibrosis generated. Furthermore, it can also be useful in guiding the application of certain preventive treatments based on the risk identified in the patient, such as, for example, adjusting the diet or apply preoperative physiotherapy, oral pharmacology, or alternative surgical techniques, that prevent the generation of fibrosis. Likewise, it allows knowing a patient's risk of suffering postoperative fibrosis before undergoing surgery.

Method of the Invention

In a first aspect, the present invention relates to an *in vitro* method of obtaining data useful for predicting the risk of a subject of suffering fibrosis, hereinafter "the method of the invention", which comprises the following steps:

(a) analyzing in an isolated biological sample from the subject, at least, one genetic polymorphism selected from the list consisting of rs679620 of the MMP3 gene, rs8032158 of the NEDD4 gene, rs12456284 of the SMAD4 gene, and any other polymorphism which is in linkage disequilibrium with said genetic polymorphisms;

(b) data from the subject of, at least, one environmental variable selected from the list consisting of: use or non-use of platelet-rich plasma in the treatment of the subject and degree of obesity;

(c) assigning a $\beta$ value to the genetic polymorphisms analyzed in step (a) according to Table 3, and assigning a $\beta$ value to the data collected from the subject in step (b) according to Table 3; and

(d) calculating a P-value of risk of suffering fibrosis by applying the algorithm:

$$P = \frac{1}{1 + e^{-(\beta_o + \beta_{1X})}}$$

wherein,

$\beta_0$ = 3.256
$\beta_{1x}$ is the sum of all the $\beta$ values assigned in step (c).

The term *"in vitro"* refers to the fact that the method of the invention is performed outside the body of the subject.

The term "fibrosis" refers to a process typical of vascularized connective tissue, involving the plasma and circulating and resident cells of the connective tissue, with an emphasis on inflammation mediators, granulocytes, macrophages, platelets, and endothelial cells, in addition to fibroblasts and myofibroblasts, among others. At the cellular level, fibrosis is characterized by the proliferation of fibroblasts, cells that are abundant in connective tissue, which secrete compounds such as collagen and proteoglycans. Similarly, myofibroblasts also play a very important role during inflammation, repair, and healing. As it is used herein, it refers to a pathological increase in the connective tissue of some organ or tissue.

The term "healing", as it is used herein, refers to a natural process whereby the body of a subject who has suffered an injury regenerates the tissues that are compromised in said injury. In said process, a series of complex biochemical phenomena that take place to repair the damage caused by the injury are carried out. The healing process of the present invention refers, but is not limited to, any type of healing of a wound caused in the human body, including wounds from postoperative processes.

In some cases, said healing process gets out of control, giving rise to fibrotic processes such as the formation of keloids. The term "keloids", as it is used herein, refers to pathological scars caused by an aberrant and excessively exuberant wound healing response. Keloids are raised scars that extend beyond the margins of an original wound and invade the normal skin surrounding the wound site. Keloids may continue to grow over time and not regress spontaneously. A keloid lesion can be considered to be made up of several different parts that may have very different biological activity from one another. In general, the central part of a mature keloid lesion (the intralesional portion) is largely acellular, while the peripheral part of the lesion (the perilesional portion) is relatively more cellular and is the site of highest angiogenic activity.

In the present invention, the expression "predicting the risk of a subject of suffering fibrosis" refers to determining or predicting the probability of a subject of suffering said condition. In the present invention, obtaining data useful for this prediction comprises the application of an algorithm that takes into account environmental and genetic variables, in particular, genetic polymorphisms.

In the present invention, the algorithm of the method of the invention is considered predictive when the AUCROC (AUC-area under curve; ROC-receiver operating characteristic) is greater than 0.5. The AUCROC is defined as the probability of correctly classifying a pair of case and control individuals, randomly selected from the population, by means of the results or values obtained when applying the algorithm thereto.

By convention, the AUCROC is comprised between 0.5 and 1: the AUCROC value of a variable is considered to be more accurate and predictive the closer it is to 1. The sensitivity of the diagnostic test is the probability of obtaining a positive result when the individual has the disease or condition, in the present invention, fibrosis. The specificity of a test indicates the probability of obtaining a negative result when the individual does not have the disease or condition, in the present invention, when the individual does not develop or suffer fibrosis.

In the present invention, the term "subject" refers to any individual susceptible of suffering fibrosis, or to any individual whose risk of suffering said condition is of interest. The terms "patient" or "subject" are used interchangeably in the present invention.

In the present invention, fibrosis can affect any organ or tissue of the subject. Preferably, the fibrosis affects a joint. More preferably, the fibrosis affects a joint, wherein the joint is selected from the list consisting of knee, ankle, hip, shoulder, and elbow.

The method of the invention furthermore allows predicting a subject's risk of suffering postoperative fibrosis prior to being subjected to a surgery.

Thus, in a preferred embodiment, alone or in combination with the other preferred embodiments, the subject is to be subjected to a surgery. In another more preferred embodiment, the surgery to which the subject is to be subjected is performed on a joint. Examples of joints include, but are not limited to, knee, ankle, hip, shoulder, and elbow. Thus, in another even more preferred embodiment, the joint is selected from the list consisting of knee, ankle, hip, shoulder, and elbow.

In another preferred embodiment of the method of the invention, alone or in combination with the other preferred embodiments, the fibrosis is postoperative fibrosis.

The term "postoperative fibrosis" refers to the fibrosis process in response to a surgery (or surgical intervention, terms used interchangeably herein) which comprises a secondary scarring process. As it is used herein, postoperative fibrosis is used to refer to a condition which comprises excessive secondary scarring caused after a surgical intervention, with more fibrous tissue being formed than necessary, causing undesired effects that can complicate the patient's recovery. Examples of undesired effects relating to said postoperative fibrosis include, but are not limited to, compression of a nerve due to excessive scarring which causes pain to the patient, affecting the patient's recovery and the quality of life of those who suffer same or restricting the range of movement of a joint after surgical intervention.

Postoperative fibrosis usually appears at the joint level, leading to the development of arthrofibrosis, with the subsequent pathological stiffening or hardening of a joint due to an exaggerated fibrotic response. This can appear in large joints such as shoulders, elbows, hip, and knees, and generally cause loss of function and immobility of said joints. In that sense, in a preferred embodiment of the method of the invention, postoperative fibrosis occurs in a joint. Preferably, the joint is selected from the list consisting of shoulder, elbow, hip, and knee.

*Step (a)* of *the method of the invention: analysis of genetic polymorphisms*

A first step of the method of the invention [step (a)] comprises analyzing in a biological sample isolated from the subject, at least, one genetic polymorphism selected from the list consisting of rs679620 of the MMP3 gene, rs8032158 of the NEDD4 gene, rs12456284 of the SMAD4 gene, and any other polymorphism which is in linkage disequilibrium with said genetic polymorphisms;
The term "genetic polymorphism" refers to a variation in the nucleotide sequence of the deoxyribonucleic acid (DNA) chain that has a frequency of at least 1% in individuals in a population. Genetic polymorphisms can be variations of one or more nucleotides. Single nucleotide polymorphisms, or SNP, generally give rise to two alleles.

The terms "polynucleotide", "nucleotide sequence", "sequence of nucleotides", "nucleic acid", and "oligonucleotide" are used interchangeably herein, and refer to a polymeric form of nucleotides of any length that may or may not be chemically or biochemically modified.

In the present invention, the term "analyze" referring to genetic polymorphism(s) refers to detecting said polymorphism(s), determining the genotype or genotypes thereof.

Genetic polymorphism rs679620 of the MMP3 gene refers to an SNP located at position 102842889 of chromosome 11 in *Homo sapiens* (GenBank accession number of chromosome 11 sequence in *Homo sapiens*:NC_000011.10). The genotypes can be homozygous for adenine (A:A), heterozygous for adenine:guanine (A:G), or homozygous for guanine (G:G).

Genetic polymorphism rs8032158 of the NEDD4 gene refers to an SNP located at position 55902679 of chromosome 15 in *Homo sapiens* (GenBank accession number of chromosome 15 sequence in *Homo sapiens:* NC_000015.10). The genotypes can be homozygous for cytosine (C:C), heterozygous for cytosine:thymine (C:T), or homozygous for thymine (T:T).

Genetic polymorphism rs12456284 of the SMAD4 gene refers to an SNP located at position 51083598 of chromosome 18 in *Homo sapiens* (GenBank accession number of chromosome 18 sequence in *Homo sapiens:* NC_000018.10). The genotypes can be homozygous for adenine (A:A), heterozygous for adenine:guanine (A:G), or homozygous for guanine (G:G).

In a preferred embodiment of the method of the invention, alone or in combination with other preferred embodiments of the method of the invention, step (a) comprises analyzing, at least, two genetic polymorphisms selected from the list consisting rs679620 of the MMP3 gene, rs8032158 of the NEDD4 gene, rs12456284 of the SMAD4 gene, and any other polymorphism which is in linkage disequilibrium with said genetic polymorphisms.

In a more preferred embodiment, alone or in combination with the other preferred embodiments, step (a) comprises analyzing genetic polymorphism rs12456284 of the SMAD4 gene and genetic polymorphism rs679620 of the MMP3 gene.

In another more preferred embodiment, alone or in combination with the other preferred embodiments, step (a) comprises analyzing genetic polymorphism rs8032158 of the NEDD4 gene and genetic polymorphism rs12456284 of the SMAD4 gene.

In another more preferred embodiment, alone or in combination with the other preferred embodiments, step (a) comprises analyzing genetic polymorphism rs8032158 of the NEDD4 gene and genetic polymorphism rs679620 of the MMP3 gene.

In another preferred embodiment, alone or in combination with the other preferred embodiments, step (a) comprises analyzing, at least, three genetic polymorphisms selected from the list consisting rs679620 of the MMP3 gene, rs8032158 of the NEDD4 gene, rs12456284 of the SMAD4 gene, and any other polymorphism which is in linkage disequilibrium with said genetic polymorphisms.

In another more preferred embodiment, alone or in combination with the other preferred embodiments, step (a) comprises analyzing three genetic polymorphisms selected from the list consisting rs8032158 of the NEDD4 gene, rs12456284 of the SMAD4 gene, rs679620 of the MMP3 gene, and any other polymorphism which is in linkage disequilibrium with said genetic polymorphisms. Even more preferably, the genetic polymorphisms are rs8032158 of the NEDD4 gene, rs12456284 of the SMAD4 gene, and rs679620 of the MMP3 gene.

In addition to the analysis of at least one, at least two, at least three, or three of genetic polymorphisms rs679620 of the MMP3 gene, rs8032158 of the NEDD4 gene, rs12456284 of the SMAD4 gene, or any other polymorphism which is in linkage disequilibrium with said genetic polymorphisms, step (a) of the method of the invention may comprise analyzing, at least, one genetic polymorphism selected from the list consisting of rs2228145 of the IL6R gene, rs9493150 of the CTGF gene, rs1800796 of the IL-6 gene, rs17563 of the BMP4 gene, and/or any other polymorphism which is in linkage disequilibrium with said genetic polymorphisms.

Genetic polymorphism rs2228145 of the IL6R gene refers to an SNP located at position 154454494 of chromosome 1 in *Homo sapiens* (GenBank accession number of chromosome 1 sequence in *Homo sapiens:* NC_000001.11). The genotypes can be homozygous for adenine (A:A), heterozygous for adenine:cytosine (A:C), or homozygous for cytosine (C:C).

Genetic polymorphism rs9493150 of the CTGF gene refers to an SNP located at position 131952851 of chromosome 6 in *Homo sapiens* (GenBank accession number of chromosome 6 sequence in *Homo sapiens:* NC_000006.12). The genotypes can be homozygous for cytosine (C:C), heterozygous for cytosine:guanine (C:G), or homozygous for guanine (G:G).

Genetic polymorphism rs1800796 of the IL-6 gene refers to an SNP located at position 22726627 of chromosome 7 in *Homo sapiens* (GenBank accession number of chromosome 7 sequence in *Homo sapiens:* NC_000007.14). The genotypes can be homozygous for cytosine (C:C), heterozygous for cytosine:guanine (C:G), or homozygous for guanine (G:G).

Genetic polymorphism rs17563 of the BMP4 gene refers to an SNP located at position 53950804 of chromosome 14 in *Homo sapiens* (GenBank accession number of chromosome 14 sequence in *Homo sapiens:* NC_000014.9). The genotypes can be homozygous for cytosine (C:C), heterozygous for cytosine:thymine (C:T), or homozygous for thymine (T:T).

Thus, in a preferred embodiment of the method of the invention, alone or in combination with the other preferred embodiments, step (a) further comprises analyzing, at least, one genetic polymorphism selected from the list consisting of rs2228145 of the IL6R gene, rs9493150 of the CTGF gene, rs1800796 of the IL-6 gene, rs17563 of the BMP4 gene, and any other polymorphism which is in linkage disequilibrium with said genetic polymorphisms.

In another preferred embodiment of the method of the invention, alone or in combination with the other preferred embodiments, step (a) further comprises analyzing, at least two, at least three, at least four, genetic polymorphisms selected from the list consisting of rs2228145 of the IL6R gene, rs9493150 of the CTGF gene, rs1800796 of the IL-6 gene, rs17563 of the BMP4 gene, and any other polymorphism which is in linkage disequilibrium with said genetic polymorphisms, including any combination thereof.

In another preferred embodiment of the method of the invention, alone or in combination with the other preferred embodiments, step (a) comprises analyzing, at least, 7 genetic polymorphisms selected from list consisting of rs2228145 of the IL6R gene, rs679620 of the MMP3 gene, rs9493150 of the CTGF gene, rs1800796 of the IL-6 gene, rs17563 of the BMP4 gene, rs8032158 of the NEDD4 gene, and rs12456284 of the SMAD4 gene, and any other polymorphism which is in linkage disequilibrium with said genetic polymorphisms, including any combination thereof.

In another more preferred embodiment of the method of the invention, alone or in combination with the other preferred embodiments, step (a) comprises analyzing the genetic polymorphisms rs2228145 of the IL6R gene, rs679620 of the MMP3 gene, rs9493150 of the CTGF gene, rs1800796 of the IL-6 gene, rs17563 of the BMP4 gene, rs8032158 of the NEDD4 gene, and rs12456284 of the SMAD4 gene.

The expression "polymorphisms of the invention" is used herein to refer to at least one genetic polymorphism selected from between polymorphisms rs679620 polymorphisms of the MMP3 gene, rs8032158 of the NEDD4 gene, rs12456284 of the SMAD4 gene, any other polymorphism which is in linkage disequilibrium with said genetic polymorphisms, and/or any combination of one or more of the preceding genetic polymorphisms with at least one genetic polymorphism selected from rs2228145 of the IL6R gene, rs9493150 of the CTGF gene, rs1800796 of the IL-6 gene, and rs17563 of the BMP4 gene, including any other polymorphisms in linkage disequilibrium with them.

In the present invention, genetic polymorphisms are SNPs, therefore the terms polymorphisms of the invention and SNPs of the invention can be used interchangeably throughout the document.

The genetic polymorphisms that can be analyzed as part of the method of the present invention and are useful in predicting the risk of a subject of suffering fibrosis are shown in Table 1.

Table 1. Genetic polymorphisms that can be included as genotypic variables within the prediction model.

| Gene Polymorphism | | Genotypes | Location |
|---|---|---|---|
| IL6R | rs2228145 | A:A | Chromosome 1:154454494 |
| | | A:C | |
| | | C:C | |
| MMP3 | rs679620 | A:A | Chromosome 11:102842889 |
| | | A:G | |
| | | G:G | |
| CTGF | rs9493150 | C:C | Chromosome 6:131952851 |
| | | C:G | |
| | | G:G | |
| IL-6 | rs1800796 | C:C | Chromosome 7:22726627 |
| | | C:G | |
| | | G:G | |
| BMP4 | rs17563 | C:C | Chromosome 14:53950804 |
| | | C:T | |
| | | T:T | |
| NEDD4 | rs8032158 | C:C | Chromosome 15:55902679 |
| | | C:T | |
| | | T:T | |
| SMAD4 | rs12456284 | A:A | Chromosome 18:51083598 |
| | | A:G | |
| | | G:G | |

On the other hand, any other polymorphism which is in linkage disequilibrium with them, can be analyzed in the present invention.

The term "linkage disequilibrium" refers to the property of some genes or DNA markers not to segregate independently, in other words, they have a recombination frequency of less than 50%. This is usually because the two *loci* involved are on the same chromosome, making it impossible to transfer them to the progeny in a random way with the separation of the chromosomes in anaphase. In the present invention, it refers to those genetic polymorphisms that, due to their physical proximity on a chromosome, appear together more frequently than would be expected by chance. A genetic polymorphism which is in linkage disequilibrium with another presents the same capacity or gives equivalent information, in the present invention, relating to predicting the risk of a subject of suffering fibrosis, since by the very definition, both polymorphisms are inherited together.

A measure of linkage disequilibrium between two genetic markers is defined as "$r^2$". Two genetic polymorphisms that have not been separated by recombination (complete linkage disequilibrium) show an $r^2=1$. In the present invention, polymorphisms which are in linkage disequilibrium with the genetic polymorphisms rs2228145 of the IL6R gene, rs679620 of the MMP3 gene, rs9493150 of the CTGF gene, rs1800796 of the IL-6 gene, rs17563 of the BMP4 gene, rs8032158 of the NEDD4 gene, and/or rs12456284 of the SMAD4 gene, preferably have a linkage disequilibrium measurement $r^2 \geq 0.8$, More preferably, a linkage disequilibrium measurement $r^2 \geq 0.9$.

The analysis of the polymorphisms of the invention can be carried out by any method known to the person skilled in the art for this purpose. For example, it can be performed by means of genotyping kits, sequencing, or amplification using PCR (polymerase chain reaction) and subsequent analysis with restriction enzymes or by means of real-time PCR. Genotyping kits may contain fluorophore-labeled oligonucleotides and may require hybridization of these nucleotides to a biological sample isolated from a subject.

Thus, in the method of the invention, the analysis of the polymorphisms of the invention of step (a) is carried out by any method known to a person skilled in the art for this purpose. In a preferred embodiment, alone or in combination with the other preferred embodiments, it is carried out by amplification, more preferably PCR amplification, and/or sequencing.

In another preferred embodiment of the method of the invention, the analysis of the polymorphisms of the invention of step (a) is carried out using a genotyping kit.

In another preferred embodiment of the method of the invention, alone or in combination with the other preferred embodiments, the analysis of the polymorphisms of the invention of step (a) comprises the use of probes and/or primers that detect and/or amplify said polymorphisms.

In a more preferred embodiment, the primers and/or probes comprise, or consist of nucleotide sequences: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, and/or any combination thereof.

SEQ ID NO: 1
CCATATTCTCCTCTTCCTCCTCTATCTTCA

SEQ ID NO: 2
CTCCAGCAACCAGGAATGTG

Sequences of SEQ ID NO:1 and SEQ ID NO: 2 correspond to the sequences of the primers necessary to amplify polymorphism rs2228145 of the IL6R gene.

SEQ ID NO: 3
GGGCAGTGGTACTGAAGAAGAAT

SEQ ID NO: 4
GGGCAGTGGTACTGAAGAAGAAG

Sequences of SEQ ID NO:3 and SEQ ID NO: 4 correspond to the sequences of the probes necessary to specifically detect polymorphism rs2228145 of the IL6R gene.

SEQ ID NO: 5
ACTTATTCTGTTAGAAATATCTAGAAAACTACTACGACCT

SEQ ID NO: 6
ACAACAGGACCACTGTCCTT

Sequences of SEQ ID NO: 5 and SEQ ID NO: 6 correspond to the sequences of the primers necessary to amplify polymorphism rs679620 of the MMP3 gene.

SEQ ID NO: 7
TCTCCTAACAAACTGTTTCACATCTTTTTT

SEQ ID NO: 8
TCTCCTAACAAACTGTTTCACATCTTTTTC

Sequences of SEQ ID NO: 7 and SEQ ID NO: 8 correspond to the sequences of the probes necessary to specifically detect polymorphism rs679620 of the MMP3 gene.

SEQ ID NO: 9
AAATGATAAATCTATAATGCATTGTTTCTAAAGCCGAT

SEQ ID NO: 10
TGTTGATGCTTCAGAGCATGG

Sequences of SEQ ID NO: 9 and 10 correspond to the sequences of the primers necessary to amplify polymorphism rs9493150 of the CTGF gene.

SEQ ID NO: 11
CATGGGTTCAAGATAAGCATATCTGTTTC

SEQ ID NO: 12
CATGGGTTCAAGATAAGCATATCTGTTTG

Sequences of SEQ ID NO: 11 and SEQ ID NO: 12 correspond to the sequences of the probes necessary to specifically detect polymorphism rs9493150 of the CTGF gene.

SEQ ID NO: 13
CTGTGTTCTGGCTCTCCCTGT

SEQ ID NO: 14
GCCTTGAAGTAACTGCACGAAATT

Sequences of SEQ ID NO: 13 and SEQ ID NO: 14 correspond to the sequences of the primers necessary to amplify polymorphism rs1800796 of the IL-6 gene.

SEQ ID NO: 15
AGGCAGTTCTACAACAGCCC

SEQ ID NO: 16
AGGCAGTTCTACAACAGCCG

Sequences of SEQ ID NO: 15 and SEQ ID NO: 16 correspond to the sequences of the probes necessary to specifically detect polymorphism rs1800796 of the IL-6 gene.

SEQ ID NO: 17
CCACCTGCTCCCGGAAGA

SEQ ID NO: 18
CGTTTCCTCTTTAACCTCAGCA

Sequences of SEQ ID NO: 17 and SEQ ID NO: 18 correspond to the sequences of the primers necessary to amplify polymorphism rs17563 of the BMP4 gene.

SEQ ID NO: 19

GCATCCCTGAGAACGAGGC

SEQ ID NO: 20
GCATCCCTGAGAACGAGGT

Sequences of SEQ ID NO: 19 and SEQ ID NO: 20 correspond to the sequences of the probes necessary to specifically detect polymorphism rs17563 of the BMP4 gene.

SEQ ID NO: 21
TGAATTTCATCCTTAAGTTCTTTTTATTTAATAAATGTTA

SEQ ID NO: 22
GTGTATGTTTGAAATTTTCCCTAACGAA

Sequences of SEQ ID NO: 21 and SEQ ID NO: 22 correspond to the sequences of the primers necessary to amplify polymorphism rs8032158 of the NEDD4 gene.

SEQ ID NO: 23
TTTAAAATATTTGGGAAAAAACTAGCTTATAATAAC

SEQ ID NO: 24
TTTAAAATATTTGGGAAAAAACTAGCTTATAATAAT

Sequences of SEQ ID NO: 23 and SEQ ID NO: 24 correspond to the sequences of the probes necessary to specifically detect polymorphism rs8032158 of the NEDD4 gene.

SEQ ID NO: 25
GGCCCAACCCAGAGCCATTA

SEQ ID NO: 26
CACACCGTTTATTTAAATGCTTTCTCA

Sequences of SEQ ID NO: 25 and SEQ ID NO: 26 correspond to the sequences of the primers necessary to amplify polymorphism rs12456284 of the SMAD4 gene.

SEQ ID NO: 27
CCAGAGCCAGTGTTCTTGTTCA

SEQ ID NO: 28
CAGAGCCAGTGTTCTTGTTCG

Sequences of SEQ ID NO: 27 and SEQ ID NO: 28 correspond to the sequences of the probes necessary to specifically detect polymorphism rs12456284 of the SMAD4 gene.

In a more preferred embodiment, the analysis of the polymorphisms carried out in step (a) comprises the use of probes which detect the polymorphisms of the invention. Preferably, the probes that detect said polymorphisms are selected from the list consisting of: SEQ ID NO: 3, SEQ ID NO: 4, SEQ ID NO: 7, SEQ ID NO: 8, SEQ ID NO: 11, SEQ ID NO: 12, SEQ ID NO: 15, SEQ ID NO: 16, SEQ ID NO: 19, SEQ ID NO: 20, SEQ ID NO: 23, SEQ ID NO: 24, SEQ ID NO: 27, SEQ ID NO: 28, and any combination thereof.

In another preferred embodiment of the method of the invention, alone or in combination with the other preferred embodiments, the analysis of the polymorphisms of the invention of step (a) comprises the use of fluorophore-labeled hybridization probes.

The term "sequencing", as it is used herein, refers to the determination of the nucleotides of a template nucleic acid and the order thereof.

The term "amplification", as it is used herein, refers to the increase in the number of copies of a template nucleic acid, where this can be carried out using fluorescent probes. In a preferred embodiment, amplification takes place by means of real-time PCR.

The term "template nucleic acid" or "template", as it is used herein, refers to a single-stranded or double-stranded nucleic acid molecule to be amplified or sequenced.

The increase in the number of copies of a template nucleic acid is carried out by means of complementary DNA synthesis under conditions that allow it. Conditions that allow complementary DNA synthesis to refer to conditions in which incorporation of nucleotides into a nascent DNA can take place by means of base complementarity with the template nucleic acid.

The conditions in which sequencing or amplification is performed generally include (a) contacting a template nucleic acid with a polymerase in a mixture further comprising a primer, a bivalent cation, (for example, Mg$^{2+}$), and nucleotides, generally, dNTPs, and at least one ddNTP, and (b) subjecting said mixture to a temperature sufficient for the polymerase to initiate the incorporation of the nucleotides into the primer by means of base complementarity with the template nucleic acid, and giving rise to a population of complementary DNA molecules of different sizes. The separation of said population of complementary DNA molecules, generally by electrophoresis, allows determining the nucleotide sequence.

The term "primer", as it used herein, refers to an oligonucleotide capable of acting as a starting point for DNA synthesis when it hybridizes with the template nucleic acid. Preferably, the primer is a deoxyribose oligonucleotide. Primers can be prepared by means of any suitable method including, for example, but not limited to, cloning and restriction of suitable sequences and direct chemical synthesis. Primers can be designed to hybridize with specific nucleotide sequences in the template nucleic acid (specific primers) or can be randomly synthesized (arbitrary primers).

The term "specific primer," as it is used herein, refers to a primer the sequence of which is complementary to a specific nucleotide sequence in the template nucleic acid to be amplified or sequenced.

The term "arbitrary primer" refers to a primer the sequence of which is randomly synthesized, and which is used to start DNA synthesis at random positions of the template nucleic acid to be amplified or sequenced. Often a population of different arbitrary primers is used. The term "arbitrary primers" refers to a set of primers the sequence of which is randomly synthesized, and which is used to start DNA synthesis at random positions of the template nucleic acid to be amplified or sequenced.

The term "hybridization," as it is used herein, refers to the pairing of two single-stranded complementary DNA molecules to give a double-stranded molecule. Preferably, complementarity is 100%. This is to say, in the region of complementarity each nucleotide of one of the two nucleic acid molecules can form hydrogen bonds with a nucleotide present in the other nucleic acid molecule. However, those of ordinary skill in the art will recognize that two nucleic acid molecules having a region with less than 100% complementarity can also hybridize.

The term "nucleotide," as it is used herein refers to an organic molecule formed by the covalent binding of a pentose, a nitrogenous base, and a phosphate group. The term nucleotide includes deoxyribonucleoside triphosphates such as, for example, but not limited to, dATP, dCTP, dITP, dUTP, dGTP, dTTP, or derivatives thereof. The term nucleotide also includes dideoxyribonucleoside triphosphates (ddNTPs), such as, for example, ddATP, ddCTP, ddGTP, ddITP, ddTTP, or derivatives thereof.

According to the present invention, a "nucleotide" or a "primer" may be labeled or tagged by techniques well known in the state of the art. Detectable labels include, for example, radioactive isotopes, fluorescent labels, chemiluminescent labels, bioluminescent labels, or enzyme labels.

The term "biological sample" in the present invention refers to any sample which allows obtaining DNA from the individual from whom said sample was obtained, and includes, but not limited to, tissues and/or biological fluids from an individual, obtained by means of any method known to a person skilled in the art that serves such purpose.

Thus, in a preferred embodiment, alone or in combination with the other preferred embodiments, the biological sample is from any tissue susceptible to DNA extraction. The biological sample could be, for example, but not limited to, a tissue or fluid sample, such as blood, plasma, serum, oral mucosa, bronchoalveolar lavage, Lymph, or ascitic fluid. In a more preferred embodiment, the biological sample is selected from the list consisting of tissue, oral mucosa, blood, plasma, serum, and lymph. Likewise, the biological sample can be, for example, but not limited to, fresh, frozen, fixed, or fixed and paraffin embedded.

*Step (b) of the method of the invention: Collecting data from the subject*

As explained above, the algorithm used in the method of the invention that is useful and allows predicting a subject's risk of suffering fibrosis is based on the use of genomic variables together with environmental variables of each subject. To that end, therefore, it is necessary collecting data related to said environmental variables of the subject.

Thus, a second step of the method of the invention [step (b)] comprises collecting data from the subject of, at least, one environmental variable selected from the list consisting of: use or non-use of platelet-rich plasma in the treatment of the subject and degree of obesity.

In a preferred embodiment of the method of the invention, alone or in combination with the other preferred embodiments, step (b) comprises collecting data from the subject, of two environmental variables, wherein the environmental variables are use or non-use of platelet-rich plasma in the treatment of the subject and degree of obesity.

In addition collecting data from the subject, of, at least, one environmental variable selected from use or non-use of platelet-rich plasma in the treatment of the subject and degree of obesity, or data of both, step (b) of the method of the invention may further comprise collecting data from the subject of, at least, one environmental variable selected from the list consisting of the area of the body susceptible to suffering fibrosis and the sex of the subject, which are environmental variables that may also be useful in predicting the risk of a subjectof suffering fibrosis.

Thus, in a preferred embodiment of the method of the invention, step (b) further comprises collecting, from the subject, data of at least one environmental variable selected from the list consisting of the area of the body susceptible to suffering fibrosis and the sex of the subject.

In a more preferred embodiment of the method of the invention, alone or in combination with the other preferred embodiments, step (b) comprises collecting data from the subject of the environmental variablesuse or non-use of platelet-rich plasma in the treatment of the subject, degree of obesity, area of the body susceptible to suffering fibrosis, and sex of the subject.

The phrase "collecting data from the subject", refers to the gathering and/or recording of data relating to environmental variables. As understood by a person skilled in the art, said data collection can be performed by means of any methodology or protocol followed by health professionals.

Table 2 shows the data which can be collected from the subject in step (b) of the method of the invention.

Table 2. Data which can be collected from the subject in step (b) of the method, and respective environmental variables thereof. The left column shows the environmental variables, while the right column shows each of the possible data to be collected from the subject for each variable.

| VARIABLE | DATA |
|---|---|
| Area of the body susceptible to suffering fibrosis | Hip |
| | Shoulder |
| | Knee |
| | Ankle |
| | Other(s) |
| Sex of the subject | Man |
| | Woman |
| Use or non-use of platelet-rich plasma (PRP) | Use |
| | Non-use |
| Degree of obesity | Body mass index (BMI) $\leq 30$ |
| | BMI >30 |
| | fat mass percentage > 25% (men) or fat mass percentage > 33% (women) |
| | fat mass percentage $\leq 25$ % (men) or fat mass percentage $\leq 33$% (women) |

Thus, in the present invention, the data collected for the degree of obesity environmental variable is BMI $\leq 30$, BMI >30, fat mass percentage > 25% (men), or fat mass percentage > 33% (women).

The data collected for the environmental variable, use or non-use of platelet-rich plasma in the treatment of the subject, is use or non-use.

The data collected for the environmental variable, sex of the subject, is man or woman.

The data collected for the environmental variable, area of the body susceptible to suffering fibrosis, is knee, ankle, hip, shoulder, elbow, or other(s).

Thus, in a more preferred embodiment, alone or in combination with the other preferred embodiments, step (b) comprises collecting data from the subject, wherein the data comprises:

- area of the body susceptible to suffering fibrosis, wherein the area of the body is selected from the list consisting of hip, shoulder, knee, ankle, or other, with the term "other" including any other part, tissue, or organ of the subject's body;
- male or female sex of the subject;
- use or non-use of platelet-rich plasma in the treatment of the subject; and/or
- degree of obesity, wherein preferably the degree of obesity comprises a body mass index of the subject greater than 30 or less than or equal to 30; and/or a percentage of fat mass greater than 25% or less than or equal to 25% in men, or a percentage of fat mass greater than 33% or less than or equal to 33% in women.

"Area of the body susceptible to suffering fibrosis" is understood herein to mean the location, tissue, organ, or part of the subject's body that may be susceptible to suffering fibrosis. Thus, in the present invention, one of the possible data collected from the subject in step (b) of the method of the invention is whether the area of the body, location, or tissue susceptible to suffering fibrosis is hip, shoulder, knee, ankle, or other, with the term "other" including any other part, tissue, or organ of the subject's body. As it is used herein, the expression "other part of the body" refers to any organ, tissue, or area of the subject's body other than the hip, shoulder, knee, ankle.

In a preferred embodiment of the method of the invention, alone or in combination with the other preferred embodiments, the area of the body susceptible to suffering fibrosis is the tissue or organ of the subject where surgery is performed.

Another of the possible data collected from the subject in this step (b) of the method of the invention is whether the sex of the subject is male or female.

In the present invention, another of the possible data collected from the subject in step (b) is the use or non-use of platelet-rich plasma in the treatment of the subject. As it is used herein, "platelet-rich plasma" (or PRP) is of the 13-00-11/24-00-11 type as described in Kon E., et al., Expert Opin Biol Ther., 20 (12):1447-1460 (2020).

In step (b) of the method of the invention, another of the possible data collected from the subject relates to the degree of obesity of the subject, with degree of obesity being understood as a body mass index greater than 30 (BMI $\leq$30), or less than or equal to 30 (BMI $\leq$30) or a percentage of fat mass greater than 25% in men or less than or equal to 25%, and greater than 33% in women or less than or equal to 33%. The "body mass index" (or BMI) of a subject, in the present invention a subject, is defined as the quotient of the subject's weight in kilograms and the square of the subject's height in meters. "Body fat percentage" can be calculated through bioelectrical impedance or skinfold plicometry.

*Step (c) of the method of the invention: assigning a $\beta$ value to the polymorphisms and data from the subject*

As already mentioned, the method of the invention takes into account genetic variables and environmental variables of each subject. A value is assigned prior to the application of the algorithm of the invention, in the present invention a $\beta$ value, to the polymorphisms analyzed in step (a), and to the collected data from the subject in step (b) of the method of the invention.

Thus, a third step of the method of the invention, step (c), comprises assigning a $\beta$ value to the genetic polymorphisms analyzed in step (a) according to Table 3, and assigning a $\beta$ value to the data collected from the subject in step (b) according to Table 3.

Table 3. Specific $\beta$ values for each possibility of the analyzed variables. In the case of the genetic polymorphisms analyzed in step (a) of the method, a $\beta$ value is assigned for each polymorphism (according to the subject's genotype for that polymorphism). In the case of data collected from the subject in step (b) of the method, a $\beta$ value is assigned to each collected data.

| Variable | Possibility | $\beta$ values |
|---|---|---|
| rs2228145 (or any polymorphism in linkage disequilibrium therewith) | A:A | -0.860 |
| | A:C | -0.860 |
| | C:C | 0.000 |
| rs679620 (or any polymorphism in linkage disequilibrium therewith) | A:A | 0.000 |
| | A:G | 0.000 |
| | G:G | 0.783 |
| rs9493150 (or any polymorphism in linkage disequilibrium therewith) | C:C | 0.000 |
| | C:G | 0.000 |
| | G:G | -0.811 |
| rs1800796 (or any polymorphism in linkage disequilibrium therewith) | C:C | -0.618 |
| | C:G | 0.000 |
| | G:G | -0.618 |

(continued)

| Variable | Possibility | β values |
|---|---|---|
| rs17563 (or any polymorphism in linkage disequilibrium therewith) | C:C | -0.690 |
| | C:T | -0.690 |
| | T:T | 0.000 |
| rs8032158 (or any polymorphism in linkage disequilibrium therewith) | C:C | 0.000 |
| | C:T | 0.000 |
| | T:T | 0.813 |
| rs12456284 (or any polymorphism in linkage disequilibrium therewith) | A:A | 0.607 |
| | A:G | 0.000 |
| | G:G | 0.607 |
| Area of the body susceptible to suffering fibrosis | Hip | -2.380 |
| | Shoulder | -2.506 |
| | Knee | -1.552 |
| | Ankle | 0.000 |
| | Other | -5.301 |
| Sex of the subject | Man | -1.219 |
| | Woman | 0.000 |
| Use or non-use of PRP in treatment | Use | 0.000 |
| | Non-use | 1.796 |
| Degree of obesity | B;I ≤30, or % of fat mass ≤ 25% (men), or ≤ 33% (women) | -1.229 |
| | BMI >30, or % of fat mass> 25% (men), or > 33% (women) | 0.000 |

Thus, in the case of the genetic polymorphisms analyzed in step (a) of the method, a β value is assigned to each polymorphism according to the genotype of the subject. In the case of data collected from the subject in step (b) of the method, a β value is assigned to each data collected.

The sum of the β values assigned in this step (c) of the method of the invention constitutes the $\beta_1 x$ value that is part of the algorithm applied in the next step of the method of the invention.

*Step (d) of the method of the invention: Calculating the P-value by means of applying an algorithm*

The next step of the method of the invention, step (d), comprises calculating a P-value of the risk of suffering fibrosis by means of applying the algorithm:

$$P = \frac{1}{1 + e^{-(\beta_o + \beta_1 X)}}$$

wherein,

$\beta_0$ = 3.256
$\beta_1 x$ is the sum of all the β values assigned in step (c).

This algorithm, hereinafter the algorithm of the invention, relates the risk of a subject of suffering fibrosis, according to previously analyzed genetic polymorphisms and data of environmental variables previously collected from the subject, through $\beta_1 x$.

$\beta_1 x$ is the sum of all the β values assigned in step (c) to each of the variables: a β value is assigned according to Table 3 to each polymorphism analyzed in step (a) of the method of the invention depending on the genotype; a β value is also assigned according to Table 3 to each data collected from the subject in step (b) of the method of the invention. The sum of all β values constitutes the β₁x value comprised in the algorithm of the invention.

The application of the algorithm of the invention allows calculating the P-value of a subject's risk of suffering fibrosis. In the present invention, the "P-value of the risk of suffering fibrosis" (also referred to as "fibrosis generation specific risk value" or simply "P-value", terms used interchangeably herein), refers to a subject's risk of suffering fibrosis. The calculated value is per unit basis and can also be interpreted in terms of percentage by multiplying the calculated value by 100. In that sense, the method of the invention allows predicting the risk of a subject of suffering fibrosis.

Kit of the invention and uses thereof

The implementation of the method of the invention is based, in addition to the collection of environmental data relating to the subject, on the analysis of genetic polymorphisms, the polymorphisms of the invention. The means used for the analysis of said genetic polymorphisms may be part of a kit.

Another aspect of the invention relates to a kit, hereinafter the "kit of the invention", comprising means for analyzing *in vitro* in an isolated biological sample from a subject, at least, three genetic polymorphisms selected from the list consisting of rs679620 of the MMP3 gene, rs8032158 of the NEDD4 gene, rs12456284 of the SMAD4 gene, and any other polymorphism which is in linkage disequilibrium with said genetic polymorphisms.

In addition, the kit of the invention may further comprise the means for analyzing *in vitro,* at least, one genetic polymorphism selected from the list consisting of rs2228145 of the IL6R gene, rs9493150 of the CTGF gene, rs1800796 of the IL-6 gene, rs17563 of the BMP4 gene, and any other polymorphism which is in linkage disequilibrium with said genetic polymorphisms. Thus, in a preferred embodiment, the kit of the invention further comprises means for analyzing *in vitro,* in the biological sample isolated from the subject, at least one genetic polymorphism selected from the list consisting of rs2228145 of the IL6R gene, rs9493150 of the CTGF gene, rs1800796 of the IL-6 gene, rs17563 of the BMP4 gene, and any other polymorphism which is in linkage disequilibrium with said genetic polymorphisms.

More preferably, the kit of the invention comprises means for analyzing *in vitro,* in a biological sample isolated from the subject, genetic polymorphisms rs2228145 of the IL6R gene, rs679620 of the MMP3 gene, rs9493150 of the CTGF gene, rs1800796 of the IL-6 gene, rs17563 of the BMP4 gene, rs8032158 of the NEDD4 gene, and rs12456284 of the SMAD4 gene.

In a preferred embodiment, alone or in combination with the other preferred embodiments, the biological sample is from any tissue susceptible to DNA extraction. The biological sample could be, for example, but not limited to, a tissue or fluid sample, such as blood, plasma, serum, oral mucosa, bronchoalveolar lavage, Lymph, or ascitic fluid. In a more preferred embodiment, the biological sample is selected from the list consisting of tissue, oral mucosa, blood, plasma, serum, and lymph.

"Means for analyzing *in vitro* genetic polymorphisms" is understood herein to mean all those reagents necessary for analyzing *in vitro* the polymorphisms of the invention (primers, probes, buffers, enzymes, coenzymes, substrates). On the other hand, the kits can include all the supports and containers necessary for its implementation and optimization (plastic tubes, plates, reagents, etc.). The kits can further contain other molecules, genes, proteins, or probes of interest, serving as positive and negative controls.

As is known to a person skilled in the art, genetic polymorphisms can be analyzed by means of techniques and methods known in the state of the art, including techniques and methods different from those presented herein. These methods and techniques have been explained in the preceding inventive aspect, and both they, as well as their preferred embodiments are applicable to the kit of the invention.

In a preferred embodiment of the kit of the invention, the means for analyzing *in vitro* the polymorphisms of the invention comprise the reagents necessary to carry out the amplification technique, preferably PCR, and/or sequencing.

In another preferred embodiment of the kit of the invention, the means for analyzing *in vitro* the polymorphisms of the invention comprise primers and/or probes which specifically detect said genetic polymorphisms.

In a more preferred embodiment of the kit of the invention, the primers and/or probes comprise, or consist of, nucleotide sequences: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, and/or any combination thereof.

More preferably, the means comprise probes that specifically detect the polymorphisms of the invention. Even more preferably, the probes that detect said polymorphisms are selected from the list consisting of: SEQ ID NO: 3, SEQ ID NO: 4, SEQ ID NO: 7, SEQ ID NO: 8, SEQ ID NO: 11, SEQ ID NO: 12, SEQ ID NO: 15, SEQ ID NO: 16, SEQ ID NO: 19, SEQ ID NO: 20, SEQ ID NO: 23, SEQ ID NO: 24, SEQ ID NO: 27, SEQ ID NO: 28, and any combination thereof.

Preferably, the kits further comprise instructions for carrying out the analysis of the polymorphisms of the invention and/or the method of the invention. These instructions may be present in the mentioned kits in different manners, one or more of which may be present in the kit. One way in which these instructions may be present is as information printed on a suitable medium or substrate, e.g., a sheet or sheets of paper on which the information is printed, in the kit packaging, in a package insert, etc. Another medium would be a computer-readable medium, for example, a CD, a USB, etc., on which the information has been recorded. Another medium that may be present is a website address that can be used over the Internet to access information at a remote site. Any suitable medium may be present in the kits.

The kit of the invention, comprising the means for analyzing *in vitro* the polymorphisms of the invention, is useful in the method of the invention. Thus, in another aspect, the invention relates to the use of the kit of the invention in the method of the invention.

The terms used to define the kit and the use of the kit of the invention have been explained for the method of the invention, and both said terms and their preferred embodiments are also applicable to the kit of the invention and its use in the method of the invention.

## DESCRIPTION OF THE FIGURES

**Figure** 1. ROC curve graph of the prediction model taking into account genetic polymorphisms rs2228145 of the IL6R gene, rs679620 of the MMP3 gene, rs9493150 of the CTGF gene, rs1800796 of the IL-6 gene, rs17563 of the BMP4 gene, rs8032158 of the NEDD4 gene, and rs12456284 of the SMAD4 gene as genetic variables, and the use or non-use of platelet-rich plasma in the treatment of the subject, degree of obesity, area of the body susceptible to suffering fibrosis, and sex of the subject as environmental variables, in which each point on the graph shows a possible cut-off point for the model in which it provides information about the specific sensitivity thereof at that point (Y axis) with respect to 1-specificity (X axis). The diagonal reference line or non-discrimination line is represented diagonally on the graph (from 0.0 to 1.1). The diagonal segments are generated by means of ties.

**Figure 2.** ROC curve graph of the prediction model taking into account polymorphism rs8032158 of the NEDD4 gene as genetic variable and the use or non-use of platelet-rich plasma as environmental variable. The diagonal segments are generated by means of ties.

**Figure 3.** ROC curve graph of the prediction model taking into account polymorphism rs12456284 of the SMAD4 gene as genetic variable and the use or non-use of platelet-rich plasma as environmental variable. The diagonal segments are generated by means of ties.

**Figure 4.** ROC curve graph of the prediction model taking into account polymorphism rs679620 of the MMP3 gene as a genetic variable and the use or non-use of platelet-rich plasma as an environmental variable. The diagonal segments are generated by means of ties.

**Figure 5.** ROC curve graph of the prediction model taking into account polymorphism rs8032158 of the NEDD4 gene as genetic variable and the degree of obesity as environmental variable. The diagonal segments are generated by means of ties.

**Figure 6.** ROC curve graph of the prediction model taking into account polymorphism rs12456284 of the SMAD4 gene as genetic variable and the degree of obesity as environmental variable. The diagonal segments are generated by means of ties.

**Figure 7.** ROC curve graph of the prediction model taking into account polymorphism rs679620 of the MMP3 gene as genetic variable and the degree of obesity as environmental variable. The diagonal segments are generated by means of ties.

**Figure 8.** ROC curve graph of the prediction model taking into account genetic polymorphisms rs12456284 of the SMAD4 gene and rs679620 of the MMP3 gene as genetic variable and the use or non-use of platelet-rich plasma as environmental variable. The diagonal segments are generated by means of ties.

**Figure 9.** ROC curve graph of the prediction model taking into account genetic polymorphisms rs8032158 of the NEDD4 gene and rs12456284 of the SMAD4 gene as genetic variable and the use or non-use of platelet-rich plasma as environmental variable. The diagonal segments are generated by means of ties.

**Figure 10.** ROC curve graph of the prediction model taking into account genetic polymorphisms rs8032158 of the NEDD4 gene and rs679620 of the MMP3 gene as genetic variable and the use or non-use of platelet-rich plasma as environmental variable. The diagonal segments are generated by means of ties.

**Figure 11.** ROC curve graph of the prediction model taking into account genetic polymorphisms rs12456284 of the SMAD4 gene and rs679620 of the MMP3 gene as genetic variable and the degree of obesity as environmental variable. The diagonal segments are generated by means of ties.

**Figure 12.** ROC curve graph of the prediction model taking into account genetic polymorphisms rs8032158 of the NEDD4 gene and rs12456284 of the SMAD4 gene as genetic variable and the degree of obesity as environmental variable. The diagonal segments are generated by means of ties.

**Figure 13.** ROC curve graph of the prediction model taking into account genetic polymorphisms rs8032158 of the NEDD4 gene and rs679620 of the MMP3 gene as genetic variable and the degree of obesity as environmental variable. The diagonal segments are generated by means of ties.

**Figure 14.** ROC curve graph of the algorithm prediction model taking into account genetic polymorphisms rs679620 of the MMP3 gene, rs8032158 of the NEDD4 gene, and rs12456284 of the SMAD4 gene as genetic variable and the use or non-use of platelet-rich plasma as environmental variable. The diagonal segments are generated by means of ties.

**Figure 15.** ROC curve graph of the prediction model taking into account genetic polymorphisms rs679620 of the MMP3 gene, rs8032158 of the NEDD4 gene, and rs12456284 of the SMAD4 gene as genetic variable and the degree of obesity as environmental variable. The diagonal segments are generated by means of ties.

**EXAMPLES**

Next, the invention will be illustrated by means of assays carried out by the inventors which demonstrate the effectiveness of the invention, including an example of the application of the method of the invention in predicting the risk of a subject of suffering fibrosis.

**1. Methodology**

A total of 240 patients who had been operated on by the traumatology team of the Arthroscopic Surgery Unit of Dr. Mikel Sanchez were selected. A buccal smear sample was taken from all of them using 4N6FLOQSwab-Life Technologies swabs. DNA of the samples was extracted by means of QIAmp DNA Mini kit (Qiagen) and quantified fluorimetrically using Qubit (Life Technologies). The resulting DNA samples were analyzed by means of a genotyping analysis of SNPs (polymorphisms rs2228145 of the IL6R gene, rs679620 of the MMP3 gene, rs9493150 of the CTGF gene, rs1800796 of the IL-6 gene, rs17563 of the BMP4 gene, rs8032158 of the NEDD4 gene, and rs12456284 of the SMAD4 gene) using the Biomark HD system (Fluidigm) methodology. Patients were categorized taking into account the following environmental factors:

- Tissue operated on: hip, shoulder, knee, ankle, or others
- male or female sex of the patient;
- use or non-use of platelet-rich plasma in the treatment of the patient. As it is used herein, "platelet-rich plasma" (or PRP) is of the 13-00-11/24-00-11 type as described in Kon E., et al., Expert Opin Biol Ther., 20 (12):1447-1460 (2020).
- degree of obesity: considering it as a patient's body mass index greater than 30, or less than or equal to 30. Weight and height were measured with the subject in underwear using a scale with a precision of 100 grams (weight) and 1 mm (height). Body mass index was calculated through the formula BMI ($kg/m^2$) = weight (kg)/(height (m))$^2$.

From data relating to environmental factors and environmental genetic polymorphisms, the inventors applied the following algorithm to obtain the fibrosis generation specific risk (referred to as P or P-value):

$$P = \frac{1}{1 + e^{-(\beta_0 + \beta_1 x)}}$$

wherein,

$\beta_0$ = 3.256

β1x is the sum of all β values assigned according to Table 3 shown herein above.

Thus, taking as an example the case of a male patient who had an injury on his right knee cruciate ligament, being an area susceptible to suffering postoperative fibrosis, for which the following data was collected:

Weight = 83kg
Height: 1.70 m
BMI= 83 / (1.70)$^2$= 28.71
Sex: male
Area susceptible to suffering fibrosis: Knee.
Use of (PRGF®-Endoret®): Yes

Furthermore, a genetic profile was made for rs2228145 of the IL6R gene, rs679620 of the MMP3 gene, rs9493150 of the CTGF gene, rs1800796 of the IL-6 gene, rs17563 of the BMP4 gene, rs8032158 of the NEDD4 gene, and rs12456284 of the SMAD4 gene, obtaining the following genetic profile:

rs2228145 = A:C
rs679620 = A:A
rs9493150 = C:G
rs1800796 = G:G
rs17563 = C:T
rs8032158 = C:C
rs12456284 = A:A

Once the data is obtained, the β value for each one was obtained, and all the β were added to obtain the $\beta_1$x value. Said value, as well as the assignment of each β value (according to Table 3 shown herein above), is represented in Table 4.

Table 4. β values for the variables collected for the patient in question.

| Variable | Possibility | Beta (β) |
|---|---|---|
| rs2228145 | A:C | -0.860 |
| rs679620 | A:A | 0.000 |
| rs9493150 | C:G | 0.000 |
| rs1800796 | G:G | -0.618 |
| rs17563 | C:T | -0.690 |
| rs8032158 | C:C | 0.000 |
| rs12456284 | A:A | 0.607 |
| AREA | Knee | -1.552 |
| SEX | Man | -1.219 |
| PRP | Yes | 0.000 |
| BMI | ≤30 | -1.229 |

$$\beta_1 X = -5.561$$

Once the value $\beta_1$x = -5.561 is obtained and taking into account that $\beta_0$= 3.256, the variables are replaced with these values in the formula:

$$P = \frac{1}{1 + e^{-(\beta_o + \beta_1 x)}}$$

obtaining as result a P=0.090, that is, a 9.00% risk of suffering fibrosis.

2. Results

Following a methodology as explained above in patients, it was applied in the algorithm of the invention, i.e.:

$$P = \frac{1}{1 + e^{-(\beta_o + \beta_1 x)}}$$

taking into account different genetic and environmental variables, with an ROC analysis being performed for each case.

2.1. ROC analysis taking into account genetic polymorphisms rs2228145 of the IL6R gene, rs679620 of the MMP3 gene, rs9493150 of the CTGF gene, rs1800796 of the IL-6 gene, rs17563 of the BMP4 gene, rs8032158 of the NEDD4 gene, and rs12456284 of the SMAD4 gene, and the environmental variables use or non-use of platelet-rich plasma in the treatment of the subject, degree of obesity, area of the body susceptible to suffering fibrosis, and sex of the subject.

ROC analysis was performed (Figure 1) by establishing a 50.90% risk of suffering fibrosis as a cut-off point to classify the subject or patient in question as having a high or low risk of developing fibrosis. Thus, if the P-value calculated for the subject is greater than 0.509, the subject is classified as having a high risk of suffering fibrosis, meanwhile if the P-value is less than 0.509, the subject is classified as having a low risk of suffering fibrosis. Taking the exemplary case of the male patient for whom P=0.090 was obtained, said value is clearly lower than the selected cut-off point of 0.509, therefore said patient has a low risk of developing fibrosis.

The model showed a sensitivity value of 74.8% and a specificity value of 73.6%. Next, a patient classification table for the"Observed" with respect to the "Predicted" using the model (with .00 being no fibrosis and 1.00 presence of fibrosis) is shown below (Table 5).

Table 5. Patient classification table[a]

| | | | Predicted | | |
| | | | Fibrosis_X | | |
| Observed | | | .00 | 1.00 | Percentage correct |
|---|---|---|---|---|---|
| Step 1 | Fibrosis_X | .00 | 89 | 32 | 73.6 |
| | | 1.00 | 30 | 89 | 74.8 |
| Overall percentage | | | | | 74.2 |

a. The cut-off value is 0.509

Table 6 shows the value of the area under the curve for the model, with an AUC (area under the curve) of 0.818 and its confidence interval of a 95% (95% CI) was 0.766-0.871.

Table 6. Area under the curve

Test result variables: Predicted probability

| | | | 95% of asymptotic confidence interval | |
| Area | Dev. Error[a] | Asymptotic significance[b] | Lower limit | Upper limit |
|---|---|---|---|---|
| .818 | .027 | .000 | .766 | .871 |

Test result variables: The predicted probability has at least one tie between the true positive state group and the true negative state group. The statistics could be biased.

a. Under the non-parametric assumption b. Null hypothesis: true area = 0.5

2.2. ROC analysis taking into account 2, 3, and 4 variables.

In addition to the ROC analysis taking into account all the genetic and environmental variables of the preceding section, ROC analyses were carried out taking into account a smaller number of variables, demonstrating that they are also useful in predicting the risk of a subject of suffering fibrosis, as shown by the results obtained and summarized in Table 7, Table 8, and Table 9.

Table 7. Summary of the results obtained from the ROC analysis (2 variables) based on the genetic and environmental variables taken into account when applying the algorithm.

| | | Variables taken into account: 1 environmental, 1 polymorphism | | | | | |
|---|---|---|---|---|---|---|---|
| | Gene | Polymorphism | % of total predicted | Cut-off value | Specificity | Sensitivity | Area of the ROC curve |
| Platelet-rich plasma (Use/-non-use) | NEDD4 | rs8032158 | 63.5 | 0.418 | 54.8 | 72.5 | 0.676 |
| | SMAD4 | rs12456284 | 61.6 | 0.395 | 42.7 | 81.0 | 0.680 |
| | MMP3 | rs679620 | 65.3 | 0.449 | 68.5 | 62.0 | 0.674 |
| Degree of obesity | NEDD4 | rs8032158 | 57.5 | 0.559 | 61.0 | 54.0 | 0.584 |
| | SMAD4 | rs12456284 | 58.5 | 0.550 | 48.8 | 68.0 | 0.597 |
| | MMP3 | rs679620 | 58.9 | 0.524 | 73.2 | 44.8 | 0.591 |

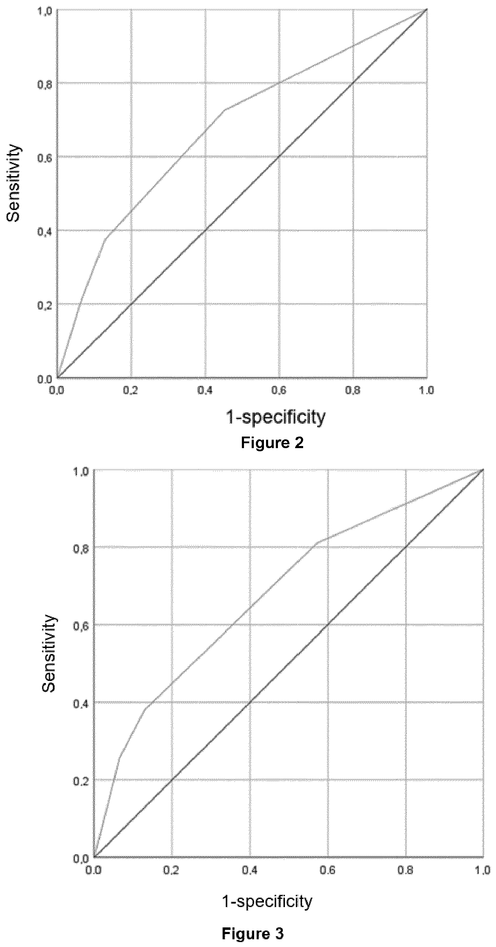

Figure 2 shows the ROC curve when applying the algorithm taking into account polymorphism rs8032158 of the NEDD4 gene as genetic variable and the use or non-use of platelet-rich plasma as environmental variable.

Figure 3 shows the ROC curve when applying the algorithm taking into account polymorphism rs12456284 of the SMAD4 gene as genetic variable and the use or non-use of platelet-rich plasma as environmental variable.

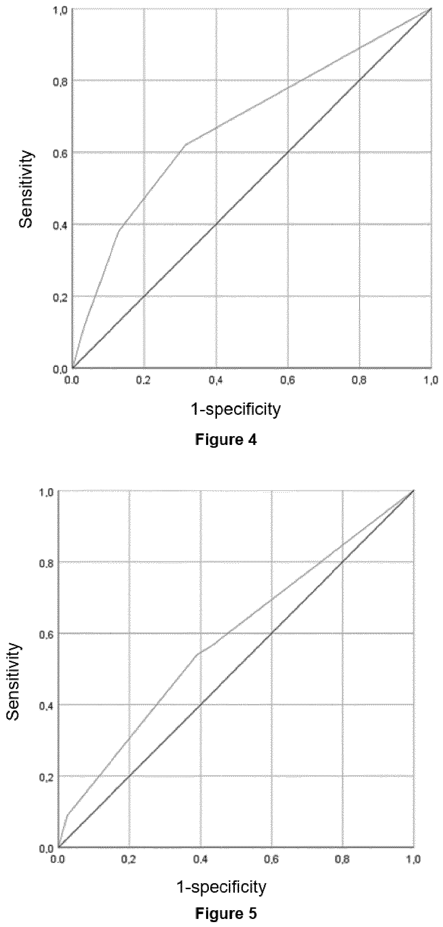

Figure 4 shows the ROC curve when applying the algorithm taking into account polymorphism rs679620 of the MMP3 gene as genetic variable and the use or non-use of platelet-rich plasma as environmental variable.

Figure 5 shows the ROC curve when applying the algorithm taking into account polymorphism rs8032158 of the NEDD4 gene as genetic variable and the degree of obesity as environmental variable.

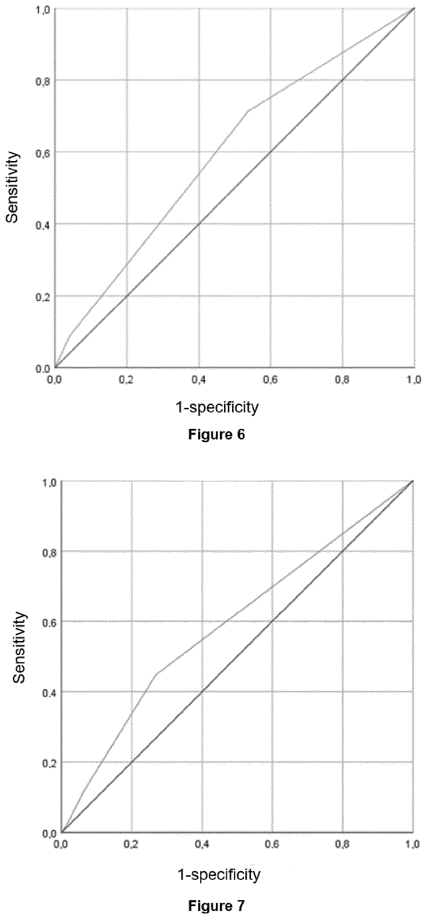

Figure 6 shows the ROC curve when applying the algorithm taking into account polymorphism rs12456284 of the SMAD4 gene as genetic variable and the degree of obesity as environmental variable.

Figure 7 shows the ROC curve when applying the algorithm taking into account polymorphism rs679620 of the MMP3 gene as genetic variable and the degree of obesity as environmental variable.

Table 8. Summary of the results obtained from the ROC analysis (3 variables) based on the genetic and environmental variables taken into account when applying the algorithm.

| | | Variables taken into account: 1 environmental, 2 polymorphisms | | | | | |
|---|---|---|---|---|---|---|---|
| | Gene | Polymorphism | % of total predicted | cut-off value | Specificity | Sensitivity | Area of the ROC curve |
| Platelet-rich plasma (Use/-non-use) | SMAD4 | rs12456284 | 66.1 | 0.516 | 75.8 | 56.2 | 0.701 |
| | MMP3 | rs679620 | | | | | |
| | NEDD4 | rs8032158 | 65.2 | 0.486 | 67.7 | 62.5 | 0.701 |
| | SMAD4 | rs12456284 | | | | | |
| | NEDD4 | rs8032158 | 65.2 | 0.455 | 68.5 | 61.7 | 0.702 |
| | MMP3 | rs679620 | | | | | |
| Degree of obesity | SMAD4 | rs12456284 | 58.9 | 0.509 | 46.3 | 71.2 | 0.628 |
| | MMP3 | rs679620 | | | | | |
| | NEDD4 | rs8032158 | 59.5 | 0.538 | 75.6 | 43.5 | 0.629 |
| | SMAD4 | rs12456284 | | | | | |
| | NEDD4 | rs8032158 | 59.1 | 0.534 | 76.4 | 41.9 | 0.628 |
| | MMP3 | rs679620 | | | | | |

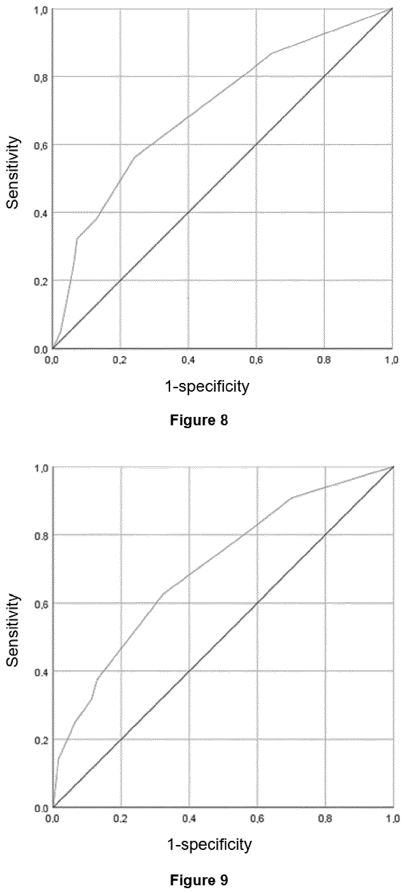

Figure 8 shows the ROC curve when applying the algorithm taking into account genetic polymorphisms rs12456284 of the SMAD4 gene and rs679620 of the MMP3 gene as genetic variables and the use or non-use of platelet-rich plasma as environmental variable.

Figure 9 shows the ROC curve when applying the algorithm taking into account genetic polymorphisms rs8032158 of the NEDD4 gene and rs12456284 of the SMAD4 gene as genetic variables and the use or non-use of platelet-rich plasma as environmental variable.

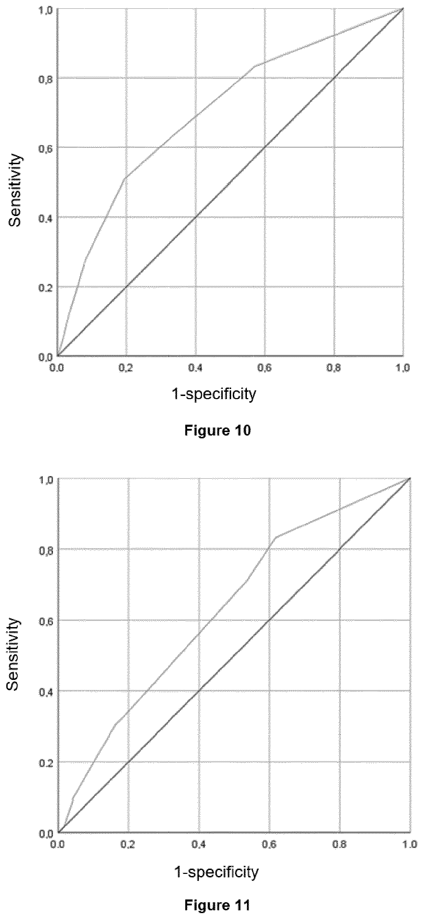

Figure 10 shows the ROC curve when applying the algorithm taking into account genetic polymorphisms rs8032158 of the NEDD4 gene and rs679620 of the MMP3 gene as genetic variables and the use or non-use of platelet-rich plasma as environmental variable.

Figure 11 shows the ROC curve when applying the algorithm taking into account genetic polymorphisms rs12456284 of the SMAD4 gene and rs679620 of the MMP3 gene as genetic variables and the degree of obesity as environmental variable.

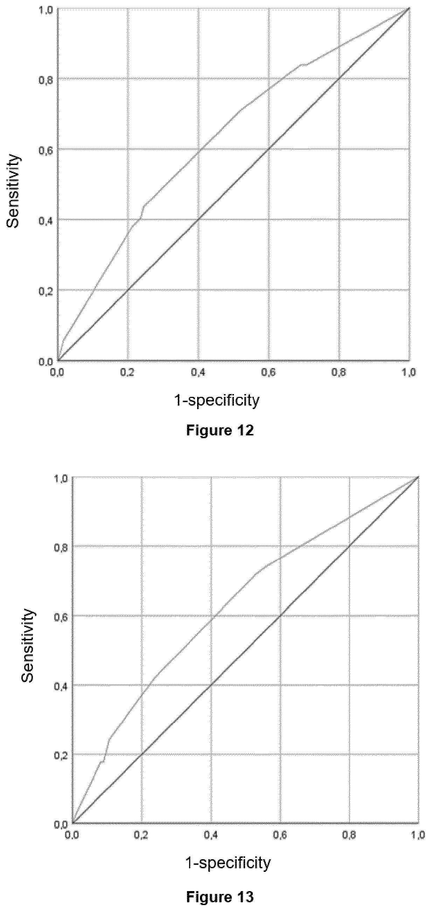

Figure 12 shows the ROC curve when applying the algorithm taking into account genetic polymorphisms rs8032158 of the NEDD4 gene and rs12456284 of the SMAD4 gene as genetic variables and the degree of obesity as environmental variable.

Figure 13 shows the ROC curve when applying the algorithm taking into account genetic polymorphisms rs8032158 of the NEDD4 gene and rs679620 of the MMP3 gene as genetic variables and the degree of obesity as environmental variable.

Table 9. Summary of the results obtained from the ROC analysis (4 variables) based on the genetic and environmental variables taken into account when applying the algorithm.

| | Variables taken into account: 1 environmental, 3 polymorphisms | | | | | | |
| --- | --- | --- | --- | --- | --- | --- | --- |
| | Gene | Polymorphism | % of total predicted | Cut-off value | Specificity | Sensitivity | Area of the ROC curve |
| Platelet-rich plasma (Use/-non-use) | NEDD4 | rs8032158 | 68.4 | 0.436 | 62.9 | 74.2 | 0.725 |
| | SMAD4 | rs12456284 | | | | | |
| | MMP3 | rs679620 | | | | | |
| Degree of obesity | NEDD4 | rs8032158 | 64.0 | 0.447 | 61.8 | 66.1 | 0.667 |
| | SMAD4 | rs12456284 | | | | | |
| | MMP3 | rs679620 | | | | | |

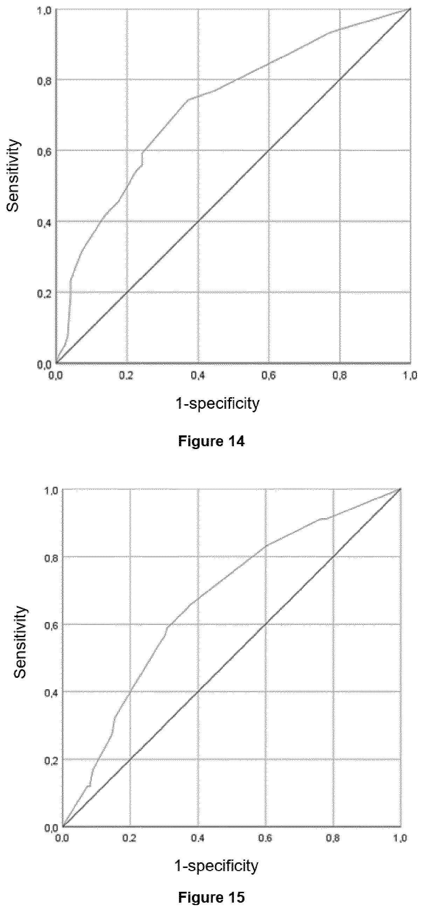

Figure 14 shows the ROC curve when applying the algorithm taking into account genetic polymorphisms rs679620 of the MMP3 gene, rs8032158 of the NEDD4 gene, and rs12456284 of the SMAD4 gene as genetic variables and the use or non-use of platelet-rich plasma as environmental variable.

Figure 15 shows the ROC curve when applying the algorithm taking into account genetic polymorphisms rs679620 of the MMP3 gene, rs8032158 of the NEDD4 gene, and rs12456284 of the SMAD4 gene as genetic variables and the degree of obesity as environmental variable.

**Claims**

**1.** An *in vitro* method of obtaining data useful for predicting the risk of a subject of suffering fibrosis, which comprises the following steps:

(a) analyzing in an isolated biological sample from the subject, at least, one genetic polymorphism selected from the list consisting of rs679620 of the MMP3 gene, rs8032158 of the NEDD4 gene, rs12456284 of the SMAD4 gene, and any other polymorphism which is in linkage disequilibrium with said genetic polymorphisms;
(b) collecting data from the subject of, at least, one environmental variable selected from the list consisting of: use or non-use of platelet-rich plasma in the treatment of the subject and degree of obesity;
(c) assigning a β value to the genetic polymorphisms analyzed in step (a) according to Table 3, and assigning a β value to the data collected from the subject in step (b) according to Table 3; and
(d) calculating a P-value of risk of suffering fibrosis, applying the algorithm:

$$P = \frac{1}{1 + e^{-(\beta_0 + \beta_1 X)}}$$

wherein,

$\beta_0 = 3.256$

$\beta_1 x$ is the sum of all the $\beta$ values assigned in step (c).

2. The method according to claim 1, wherein the step (a) comprises analyzing, at least, two genetic polymorphisms selected from the list consisting of rs679620 of the MMP3 gene, rs8032158 of the NEDD4 gene, rs12456284 of the SMAD4 gene, and any other polymorphism which is in linkage disequilibrium with said genetic polymorphisms.

3. The method according to claim 2, wherein the step (a) comprises analyzing, at least, three genetic polymorphisms selected from the list consisting of rs679620 of the MMP3 gene, rs8032158 of the NEDD4 gene, rs12456284 of the SMAD4 gene, and any other polymorphism which is in linkage disequilibrium with said genetic polymorphisms.

4. The method according to claim 3, wherein the step (a) comprises analyzing three genetic polymorphisms selected from the list consisting rs8032158 of the NEDD4 gene, rs12456284 of the SMAD4 gene, rs679620 of the MMP3 gene, and any other polymorphism which is in linkage disequilibrium with said genetic polymorphisms.

5. The method according to claim 4, wherein the genetic polymorphisms are rs8032158 of the NEDD4 gene, rs12456284 of the SMAD4 gene, and rs679620 of the MMP3 gene.

6. The method according to any one of claims 1 to 5, wherein the step (a) further comprises analyzing, at least, one genetic polymorphism selected from the list consisting of rs2228145 of the IL6R gene, rs9493150 of the CTGF gene, rs1800796 of the IL-6 gene, rs17563 of the BMP4 gene, and any other polymorphism which is in linkage disequilibrium with said genetic polymorphisms.

7. The method according to any one of claims 1 to 6, wherein the step (a) comprises analyzing the genetic polymorphisms rs2228145 of the IL6R gene, rs679620 of the MMP3 gene, rs9493150 of the CTGF gene, rs1800796 of the IL-6 gene, rs17563 of the BMP4 gene, rs8032158 of the NEDD4 gene, and rs12456284 of the SMAD4 gene.

8. The method according to any one of claims 1 to 7, wherein the step (b) comprises collecting from the subject data of two environmental variables, wherein the environmental variables are use or non-use of platelet-rich plasma in the treatment of the subject and degree of obesity.

9. The method according to any one of claims 1 to 8, wherein the step (b) further comprises collecting from the subject data of, at least, one environmental variable selected from the list consisting of the area of the body susceptible to suffering fibrosis and the sex of the subject.

10. The method according to any one of claims 1 to 9, wherein the step (b) comprises collecting from the subject data of the environmental variablesuse or non-use of platelet-rich plasma in the treatment of the subject, degree of obesity, area of the body susceptible to suffering fibrosis and sex of the subject.

11. The method according to any one of claims 1 to 10, wherein the fibrosis affects a joint.

12. The method according to claim 11, wherein the joint is selected from the list consisting of knee, ankle, hip, shoulder and elbow.

13. The method according to any one of claims 1 to 12, wherein the fibrosis is postoperative fibrosis.

14. The method according to any one of claims 1 to 13, wherein the subject is to be subjected to a surgery.

15. The method according to claim 14, wherein the surgery is performed on a joint.

16. The method according to claim 15, wherein the joint is selected from the list consisting of knee, ankle, hip, shoulder and elbow.

17. The method according to any one of claims 1 to 16, wherein the biological sample is from any tissue susceptible to DNA extraction.

18. The method according to any one of claims 1 to 17, wherein the analysis of the polymorphisms carried out in step (a) comprises the use of probes that detect said polymorphisms.

19. The method according to claim 18, wherein the probes are selected from the list consisting of: SEQ ID NO: 3, SEQ ID NO: 4, SEQ ID NO: 7, SEQ ID NO: 8, SEQ ID NO: 11, SEQ ID NO: 12, SEQ ID NO: 15, SEQ ID NO: 16, SEQ ID NO: 19, SEQ ID NO: 20, SEQ ID NO: 23, SEQ ID NO: 24, SEQ ID NO: 27, SEQ ID NO: 28, and any combination thereof.

20. The method according to any one of claims 1 to 19, wherein the analysis of polymorphisms is carried out in step (a) by PCR and/or sequencing.

21. A kit comprising means for analyzing *in vitro* in an isolated biological sample from a subject, at least, three genetic polymorphisms selected from the list consisting of rs679620 of the MMP3 gene, rs8032158 of the NEDD4 gene, rs12456284 of the SMAD4 gene, and any other polymorphism which is in linkage disequilibrium with said genetic polymorphisms.

22. The kit according to claim 21, which further comprises means for analyzing *in vitro* in the isolated biological sample from the subject, at least, one genetic polymorphism selected from the list consisting of rs2228145 of the IL6R gene, rs9493150 of the CTGF gene, rs1800796 of the IL-6 gene, rs17563 of the BMP4 gene, and any other polymorphism which is in linkage disequilibrium with said genetic polymorphisms.

23. The kit according to claim 22, comprising means for analyzing *in vitro* in an isolated biological sample from the subject, the genetic polymorphisms rs2228145 of the IL6R gene, rs679620 of the MMP3 gene, rs9493150 of the CTGF gene, rs1800796 of the IL-6 gene, rs17563 of the BMP4 gene, rs8032158 of the NEDD4 gene and rs12456284 of the SMAD4 gene.

24. Use of a kit according to any one of claims 21 to 23, in the method according to any one of claims 1 to 20.

Figure 1

1-specificity

Figure 2

1-specificity

Figure 3

Figure 4

Figure 5

**Figure 6**

**Figure 7**

**Figure 8**

**Figure 9**

Figure 10

Figure 11

Figure 12

Figure 13

Figure 14

Figure 15

# INFORME DE BÚSQUEDA INTERNACIONAL

| Solicitud internacional Nº |
| --- |
| **PCT/ES2023/070378** |

| | |
| --- | --- |
| **A.** | **CLASIFICACIÓN DEL OBJETO DE LA SOLICITUD** |

*C12Q 1/6883*(2018.01)i

De acuerdo con la Clasificación Internacional de Patentes (CIP) o según la clasificación nacional y CIP.

| | |
| --- | --- |
| **B.** | **SECTORES COMPRENDIDOS POR LA BÚSQUEDA** |

Documentación mínima buscada (sistema de clasificación seguido de los símbolos de clasificación)

C12Q

Otra documentación consultada, además de la documentación mínima, en la medida en que tales documentos formen parte de los sectores comprendidos por la búsqueda

Bases de datos electrónicas consultadas durante la búsqueda internacional (nombre de la base de datos y, si es posible, términos de búsqueda utilizados)

EPO-Internal, WPI Data

| | |
| --- | --- |
| **C.** | **DOCUMENTOS CONSIDERADOS RELEVANTES** |

| Categoría* | Documentos citados, con indicación, si procede, de las partes relevantes | Relevante para las reivindicaciones nº |
| --- | --- | --- |
| X | NAKASHIMA MITSUKO ET AL. "A genome-wide association study identifies four susceptibility loci for keloid in the Japanese population" *NATURE GENETICS, NATURE PUBLISHING GROUP US, NEW YORK,* Vol. 42, No. 9, 31 agosto 2010 (2010-08-31), página 768, [recuperado el 2010-08-15] DOI: 10.1038/NG.645 ISSN: 1061-4036, XP009530513 resumen página 768, columna derecha, párrafo 2 | 1-24 |
| X | EP 3425056 A1 (GENEPRED BIOTECHNOLOGIES [FR]; INST NAT SANTE RECH MED [FR] ET AL.) 09 enero 2019 (2019-01-09) reivindicaciones 1-9 | 1-20, 24 |

☑ En la continuación del Recuadro C se relacionan otros documentos     ☑ Los documentos de familias de patentes se indican en el Anexo

| | |
| --- | --- |
| \* Categorías especiales de documentos citados: | "T" documento ulterior publicado con posterioridad a la fecha de presentación internacional o de prioridad que no pertenece al estado de la técnica pertinente pero que se cita por permitir la comprensión del principio o teoría que constituye la base de la invención. |
| "A" documento que define el estado general de la técnica no considerado como particularmente relevante. | |
| "E" solicitud de patente o patente anterior pero publicada en la fecha de presentación internacional o en fecha posterior. | "X" documento particularmente relevante; la invención reivindicada no puede considerarse nueva o que implique una actividad inventiva por referencia al documento aisladamente considerado. |
| "L" documento que puede plantear dudas sobre una reivindicación de prioridad o que se cita para determinar la fecha de publicación de otra cita o por una razón especial (como la indicada). | "Y" documento particularmente relevante; la invención reivindicada no puede considerarse que implique una actividad inventiva cuando el documento se asocia a otro u otros documentos de la misma naturaleza, cuya combinación resulta evidente para un experto en la materia. |
| "O" documento que se refiere a una divulgación oral, a una utilización, a una exposición o a cualquier otro medio. | |
| "P" documento publicado antes de la fecha de presentación internacional pero con posterioridad a la fecha de prioridad reivindicada. | "&" documento que forma parte de la misma familia de patentes. |

| Fecha en que se ha concluido efectivamente la búsqueda internacional | Fecha de expedición del informe de búsqueda internacional |
| --- | --- |
| **27 septiembre 2023** | **09 octubre 2023** |

| Nombre y dirección postal de la Administración encargada de la Funcionario autorizado búsqueda internacional | Funcionario autorizado |
| --- | --- |
| **European Patent Office** **p.b. 5818, Patentlaan 2, 2280 HV Rijswijk** **Países Bajos** Nº de teléfono: **(+31-70)340-2040** Nº de fax: **(+31-70)340-3016** | **Ulbrecht, Matthias** Nº de teléfono: |

Formulario PCT/ISA/210 (segunda hoja) (Enero 2015)

**INFORME DE BÚSQUEDA INTERNACIONAL**

| | Solicitud internacional N° |
|---|---|
| | **PCT/ES2023/070378** |

**C.     DOCUMENTOS CONSIDERADOS RELEVANTES**

| Categoría* | Documentos citados, con indicación, si procede, de las partes relevantes | Relevante para las reivindicaciones n° |
|---|---|---|
| A | DAGNEAUX LOUIS ET AL. "Human Fibrosis: Is There Evidence for a Genetic Predisposition in Musculoskeletal Tissues?" *THE JOURNAL OF ARTHROPLASTY, ELSEVIER, AMSTERDAM, NL,* Vol. 35, No. 11, 04 junio 2020 (2020-06-04), páginas 3343-3352, [recuperado el 2020-06-04] DOI: 10.1016/J.ARTH.2020.05.070 ISSN: 0883-5403, XP086292548 todo el documento | 1-24 |

**INFORME DE BÚSQUEDA INTERNACIONAL**

| Solicitud internacional N° |
| --- |
| **PCT/ES2023/070378** |

---

**Recuadro I    Secuencia(s) de nucleótidos y/o de aminoácidos (continuación del punto 1.c de la primera hoja)**

---

1.  En lo que se refiere a **las secuencias de nucleótidos y/o de aminoácidos** divulgadas en la solicitud internacional y necesarias para la invención reivindicada, la búsqueda se ha llevado a cabo sobre la base de una lista de secuencias presentada o entregada:

    a.  ☐ que forma parte de la solicitud internacional tal y como se presentó:

        ☐ en formato de archivo de texto según Anexo C/ST.25.

        ☐ en formato papel o en formato de archivo de imagen.

    b.  ☐ presentada junto con la solicitud internacional de acuerdo a la Regla 13*ter*.1.a) del PCT exclusivamente a los fines de la búsqueda, en formato de archivo de texto según Anexo C/ST.25.

    c.  ☐ Presentada con posterioridad a la fecha de presentación exclusivamente a los fines de la búsqueda internacional:

        ☐ en formato de archivo de texto según Anexo C/ST.25 (Regla 13*ter*.1.a)).

        ☐ en formato papel o en formato de archivo de imagen (Regla 13*ter*.1.b) e Instrucciones Administrativas, sección 713).

2.  ☐ Además, en caso de que se haya presentado más de una versión o copia de una lista de secuencias, se ha entregado la declaración requerida de que la información contenida en las copias subsiguientes o adicionales es idéntica a la que formaba parte de la solicitud tal y como se presentó o no va más allá de lo presentado inicialmente

3.  Comentarios adicionales:

# EP 4 538 391 A1

**INFORME DE BÚSQUEDA INTERNACIONAL**
**Información relativa a miembros de familias de patentes**

Solicitud internacional N°

**PCT/ES2023/070378**

| Documento de patente citado en al informe de búsqueda | Fecha de publicación (día/mes/año) | Miembro(s) de la familia de patentes | Fecha de publicación (día/mes/año) |
|---|---|---|---|
| EP    3425056    A1 | 09 enero 2019 | EP    3425056    A1 | 09 enero 2019 |
| | | EP    3649254    A1 | 13 mayo 2020 |
| | | WO    2019008137    A1 | 10 enero 2019 |

Formulario PCT/ISA/210 (anexo_familia de patentes) (Enero 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2428583 A1
- ES 2422874 B1
- CA 2805267 A1

**Non-patent literature cited in the description**

- **KON E et al.** *Expert Opin Biol Ther.*, 2020, vol. 20 (12), 1447-1460